(19) Europäisches Patentamt
European Patent Office
Office européen des brevets

(11) Veröffentlichungsnummer: **0 311 565**
**A1**

(12) **EUROPÄISCHE PATENTANMELDUNG**

(21) Anmeldenummer: **88810666.3**

(22) Anmeldetag: **28.09.88**

(51) Int. Cl.[4]: **C 07 C 69/743**
C 07 C 43/295, A 01 N 53/00

(30) Priorität: **06.10.87 CH 3900/87**
**27.07.88 CH 2851/88**

(43) Veröffentlichungstag der Anmeldung:
**12.04.89 Patentblatt 89/15**

(84) Benannte Vertragsstaaten:
**AT BE CH DE FR GB IT LI NL**

(71) Anmelder: **CIBA-GEIGY AG**
**Klybeckstrasse 141**
**CH-4002 Basel (CH)**

(72) Erfinder: **Gsell, Laurenz, Dr.**
**Maiengasse 56**
**CH-4056 Basel (CH)**

**Farooq, Saleem, Dr.**
**Kirchackerstrasse 27**
**CH-4422 Arisdorf (CH)**

(54) **Schädlingsbekämpfungsmittel.**

(57) Neue Cyclopropancarbonsäureester der Formel

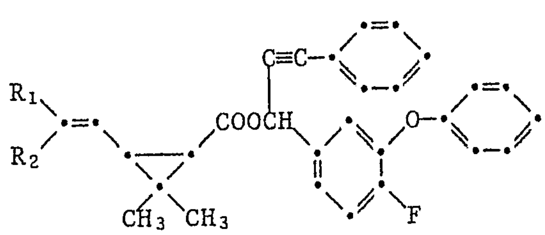

worin
$R_1$ und $R_2$ je F, Cl oder Br und falls $R_1$ Cl $R_2$ auch $CF_3$ bedeuten.
Verfahren zur Herstellung dieser Verbindungen sowie diese enthaltende Mittel und ihre Verwendung in der Schädlingsbekämpfung.

EP 0 311 565 A1

**Beschreibung**

## Schädlingsbekämpfungsmittel

Die vorliegende Erfindung betrifft neue, insektizid wirksame Cyclopropancarbonsäureester, Verfahren zu ihrer Herstellung und ihre Verwendung in der Schädlingsbekämpfung.

Die erfindungsgemässen Cyclopropancarbonsäureester haben die Formel I

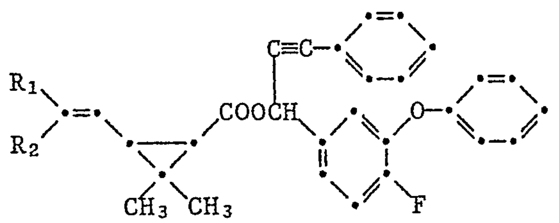

(I)

worin

$R_1$ und $R_2$ je Cl, F oder Br und falls $R_1$ Cl $R_2$ auoh $CF_3$ bedeuten.

Bevorzugt ist als Insektizid das Isomerengemisch 2,2-Dimethyl-3-cis-(2′, 2′-dichlorvinyl)-cyclopropan-1R-carbonsäure-3-phenoxy-4-fluor-α(RS)-(2′-phenyl-äthinyl)-benzylester.

Insbesondere bevorzugt sind die beiden Diastereomeren dieses Gemisches, das 2,2-Dimethyl-3-cis-(2′,2′-dichlorvinyl)-cyclopropan-1R-carbonsäure-3-phenoxy-4-fluor-α(+)-(2′-phenyl-äthinyl)-benzylester und das 2,2-Dimethyl-3-cis-(2′,2′-dichlorvinyl)-cyclopropan-1R-carbonsäure-3-phenoxy-4-fluor-α(-)-(2′-phenyl-äthinyl)-benzylester.

Aus der EP-A 190096 ist bekannt, dass sich der Ester der Formel

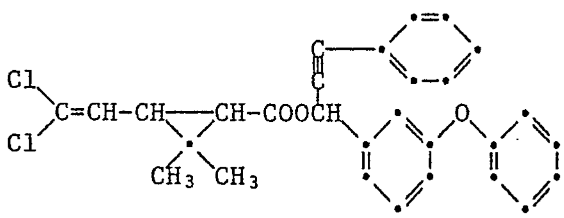

zum Schützen von keratinhaltigem Material gegen Befall durch Keratinschädlinge eignet.

Es ist ferner aus der EP-A 57384 bekannt, dass 4-Fluor-3-halophenoxybenzylester der Formel

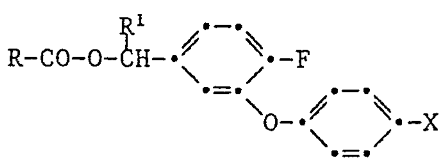

in welcher

R für einen in der Säurekomponente von Pyrethroiden verwendbaren Rest steht,

$R_1$ für Wasserstoff, Cyano oder für einen gegebenenfalls halogen-substituierten Rest aus der Reihe Alkyl, Alkenyl und Alkinyl - jeweils mit bis zu 3 Kohlenstoffatomen - steht und

X für Chlor oder Brom steht,

insektizid wirksam sind.

Es wurde nun gefunden, dass die Verbindungen der Formel I neben ihrer insektiziden Wirksamkeit eine, gegenüber dem Stand der Technik, bessere Fischverträglichkeit aufweisen.

Die Verbindungen der Formel I können durch Umsetzung eines 3-cis-1R-Cyclopropancarbonsäurehalogenids der Formel II

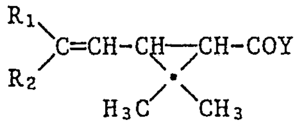

(II)

worin $R_1$ und $R_2$ die für Formel I angegebene Bedeutung haben und Y Halogen bedeutet, mit 3-Phenoxy-4-fluor-α-phenyläthinyl-benzylalkohol der Formel III

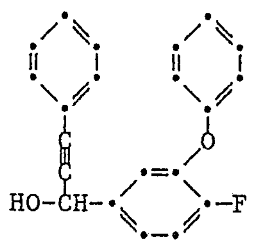

(III),

in Gegenwart einer Base hergestellt werden.

Das Zwischenprodukt der Formel III ist neu und ebenfalls Gegenstand der vorliegenden Anmeldung. Seine Herstellung erfolgt z.B. durch Umsetzung von Phenylacetylen mit Alkylmagnesiumbromid und mit 3-Phenoxy-4-fluor-benzaldehyd. Die Ausgangsprodukte dieser Reaktionsstufe sind allgemein bekannt.

Die Cyclopropancarbonsäurehalogenide der Formel II sind aus der Literatur ebenfalls bekannt.

Die Verbindungen der Formel I eignen sich zur Bekämpfung von verschiedenartigen Schädlingen an Tieren und Pflanzen. So können sie zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Acarina eingesetzt werden.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwoll- und Reiskulturen (z.B. gegen Nephotettix cinticeps, Spodoptera littoralis, Heliothis virescens, Chilo suppressalis und Laodelphax) sowie Gemüse- und Obstkulturen (z.B. gegen Leptinotarsa decemlineata, Myzus persicae, Laspeyresia pomonella und Adoxophyes reticulana) und von Bodeninsekten (z.B. Aulacophora femoralis, Chortophila brassicae, Diabrotica balteata, Pachnoda savigni und Scotia ypsilon).

Wirkstoffe der Formel I zeigen auch eine sehr günstige Wirkung gegen Fliegen, wie z.B. Musca domestica und Mückenlarven.

Die gute pestizide Wirkung der erfindungsgemässen Verbindungen entspricht einer Abtötungsrate (Mortalität) von mindestens 50-60 % der erwähnten Schädlinge.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert. welche einen synergistischen oder verstärkenden Effekt auf Pyrethroide ausüben. Beispiele solcher Verbindungen sind u.a. Piperonylbutoxid, Propionyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithionate, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahen wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise herge stellt. z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone

wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetischen oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäure von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglycoläther, Polypropylen-PolyäthylenoxidAddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp., Ridgewood, New Jersey, 1979;

Dr. Helmut Stache "Tensid Taschenbuch", Carl Hanser Verlag München/Wien 1981

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren. Entschäumer. Viskositätsregulatoren, Bindemittel Haftmittel sowie Dünger oder andere Wirkstoffe zur Erzielung spezieller Effekte enthalten.

<u>Beispiele:</u>

Herstellung der Verbindungen der Formeln I und III.

H.1 Herstellung des 3-Phenoxy-4-fluoro-α-phenyl-äthinyl-benzylalkohols

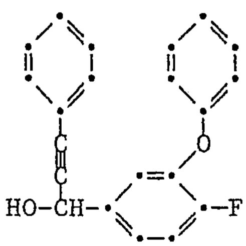

(III)

Zu einer Lösung von 66 ml Phenylacetylen (0,61 Mol) in 250 ml Tetrahydrofuran wird eine Aethylmagnesiumbromidlösung (aus 14,6 g Mg-Spähne; 50,6 ml Aethylbromid in 250 ml Tetrahydrofuran) bei 0-5° C innerhalb 3 Stunden zugetropft und eine Stunde nachgerührt. Anschliessend werden bei 0-5° C 108,1 g 3-Phenoxy-4-fluoro-benzaldehyd während 2 Stunden in die Lösung getropft und das Reaktionsgut wird 14 Stunden lang bei Raumtemperatur gerührt. Nach Zugabe von Eis wird das Reaktionsgemisch bei 5-7° C mit 100 ml konz. HCl versetzt, die wässrige Phase wird abgetrennt, mit Aether extrahiert, die vereinigte organische Phase wird mit Sole gewaschen und mit Natriumsulfat getrocknet. Nach Verdampfen des Lösungsmittels wird der Rückstand durch Chromatographie mittels Kieselgel und Essigester/Hexan = 1/3 gereinigt. Die Titelverbindung resultiert als Oel; Brechungsindex $n_D^{23}$ = 1,5971.

H.2 Herstellung des 2,2-Dimethyl-3-cis-(2′,2′-dichlorvinyl)-cyclopropan-1R-carbonsäure-3-phenoxy-4-fluor-α(RS)-(2′-phenyläthinyl)-benzylesters

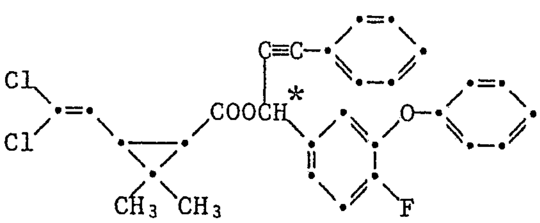

(I)

Zu 2,3 g 2,2-Dimethyl-3-cis-(2′,2′-dichlorvinyl)-cyclopropan-1R-carbonsäurechlorid (0,01 Mol), 4,8 g 3-Phenoxy-4-fluoro-α-phenyläthinyl-benzylalkohol (0,015 Mol) in 30 ml Toluol und 50 mg 4-Dimethylaminopyridin werden bei 0-5° C unter Rühren 2,8 ml Triäthylamin (0,02 Mol) gegeben und das Reaktionsgut wird 7 Std. bei 60-70° C gehalten. Nach Verdampfen des Lösungsmittels wird der Rückstand in Methylenchlorid gelöst, die Lösung mit 2n Salzsäure, dann mit Wasser, schliesslich mit Sole gewaschen, über Natriumsulfat getrocknet und eingedampft. Nach chromatographischer Reinigung des Reaktionsgemisches in toluolischer Lösung an Kieselgel, resultiert die Titelverbindung (Verb. Nr. 1.1, Tabelle 1) als gelbes Oel; Brechungsindex $n_D^{26}$ = 1.5813.

In Analogie zum Beispiel H2 werden folgende Verbindungen der Formel I hergestellt:

Tabelle 1

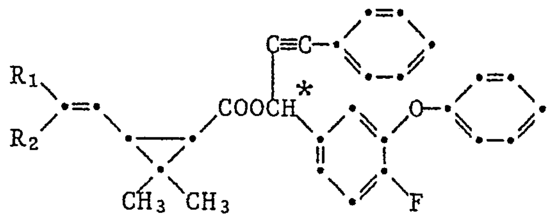

(I)

| Nr. | $R_1$ | $R_2$ | Stereochemie | Physik Daten |
|---|---|---|---|---|
| 1.1 | Cl | Cl | 1-R-cis, α-RS | Oel, $n_D^{25}$ = 1.5826 |
| 1.2 | Cl | Cl | cis, α-RS | Oel, $n_D^{24,5}$ = 1.5849 |
| 1.3 | Cl | Cl | trans, α-RS | Oel, $n_D^{25}$ = 1.5805 |
| 1.4 | $CF_3$ | Cl | cis, α-RS | Oel, $n_D^{24}$ = 1.5558 |
| 1.5 | Br | Br | Gemisch cis/trans | Oel, $n_D^{25}$ = 1.5859 |
| 1.6 | F | F | Gemisch cis/trans | Oel, $n_D^{25}$ = 1.5611 |
| 1.7 | Cl | Cl | Gemisch cis/trans = 85/15 | Oel, $n_D^{24,5}$ = 1.5894 |

H.3 Trennung der Diastereoisomeren aus H.2, Verbindung 1.1, Tabelle 1

Aus dem unter H.2 beschriebenen Reaktionsprodukt, das ein Isomerengemisch ist, können die beiden Isomeren wie folgt isoliert werden:

Nach mehrtägigem Stehen kristallisiert das nach Beispiel H.2 resultierende Oel aus. Das Kristallisat wird aus Aether/Pentan-Gemisch umkristallisiert. Smp. 134-136°C (Verbindung 2.1) $[\alpha]_D^{20}$ = -36.7° ± 0.2.

Aus der Mutterlauge wird das isomere Produkt (Verbindung 2.2) isoliert. Smp. 71-73°. $[\alpha]_D^{20}$ = + 19,1° ± 0,2.

Formulierungsbeispiele

F.1 Emulsions-Konzentrate

| | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6% |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

F.2 Lösungen

| | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190° C) | - | - | 94 % | - |

Die Lösungen sind zur Anwendung in Form kleinster Tropfen geeignet.

F.3 Granulate

| | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst. auf den Träger aufgesprüht und das Lösungsmittel anschliessend im Vakuum abgedampft.

F.4 Stäubemittel

| | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.
Biologische Beispiele

B.1: Frassgift-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend jeweils 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 2, 4 und 8 Tage nach der Behandlung.

Verbindungen Nr. 1.1, 1.2, 1.3, 1.4 gemäss Tabelle 1 und Nr. 2.1 und 2.2 gemäss Beispiel H.3 zeigen gute Wirkungen in diesem Test.

B.2 Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Verbindungen Nr. 1.1, 1.2, 1.3, 1.4 gemäss Tabelle 1 und Nr. 2.1 und 2.2 gemäss Beispiel H.3 zeigen gute Wirkungen in diesem Test.

B.3 Wirkung gegen Zecken: Abtötungwirkung bei verschiedenen Entwicklungsstadien

Als Testobjekte werden Larven (ca. 50), Nymphen (ca. 25) oder Imagines (ca. 10) der Zeckenart Boophilus microplus verwendet. Die Testtiere werden für kurze Zeit in wässrige Emulsionen bzw. Lösungen der Salze der zu untersuchenden Substanzen von bestimmter Konzentration getaucht. Die in Teströhrchen befindlichen Emulsionen bzw. Lösungen werden dann mit Watte aufgenommen und die benetzten Testtiere in den so kontaminierten Röhrchen belassen. Die Auswertung erfolgt für Larven nach 3 Tagen und für Nymphen und Imagines nach 14 Tagen. Es wird die minimale Substanzkonzentration ermittelt, die die 100%ige Abtötung ($LD_{100}$) bewirkt, ausgedrückt in ppm Wirksubstanz bezogen auf das Total von Emulsion oder Lösung.

Verbindungen Nr. 1.1, 1.2, 1.6, 1.7 gemäss Tabelle 1 und Nr. 2.1 und 2.2 gemäss Beispiel H.3 zeigen eine gute Wirksamkeit.

B.4 Wirkung gegen Dermanyssus gallinae

In einem nach oben offenen Glasbehälter werden 2 bis 3 ml einer 100 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt und in Prozenten angegeben.

Verbindungen Nr. 1.1, 1.7 gemäss Tabelle 1 und 2.1 und 2.2 gemäss Beispiel H.3 zeigen 80-100%ige Wirkung (Mortalität).

B.5 Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen Nr. 1.1 gemäss Tabelle 1 und 2.1 und 2.2 gemäss Beispiel H.3 zeigen in diesem Test gute Wirkung gegen Lucilia sericata.

B.6 Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung der Abtötungsrate festgestellt.

Verbindungen Nr. 1.1, 1.3, 1.4, 1.6 gemäss Tabelle 1 und 2.1 und 2.2 gemäss Beispiel H.3 zeigen in diesem Test gute Wirkung.

B.7 Wirkung gegen Blattella germanica

In eine Petrischale von 10 cm Durchmesser wird so viel einer 0,1%igen acetonischen Lösung des Wirkstoffes gegeben, dass die Menge einer Aufwandmenge von 2 g/m$^2$ entspricht. Wenn das Lösungsmittel verdunstet ist, werden 20 Blattella germanica Nymphen (letztes Nymphenstadium) in die so vorbereitete Schale gegeben und 2 Stunden lang der Wirkung der Testsubstanz ausgesetzt. Dann werden die Nymphen mit $CO_2$ narkotisiert, in eine frische Petrischale gebracht und im Dunkeln bei 25°C und 50 bis 70 % Luftfeuchtigkeit gehalten. Nach 48 Stunden wird die insektizide Wirkung durch Ermittlung der Abtötungsrate bestimmt.

Die Verbindung Nr. 1.3 gemäss Tabelle 1 zeigt gute Wirkung im obigen Test.

**Patentansprüche**

1. Cyclopropancarbonsäureester der Formel I

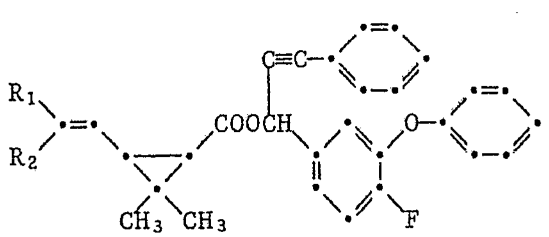

(I)

worin

$R_1$ und $R_2$ je F, Cl oder Br und falls $R_1$ Cl $R_2$ auch $CF_3$ bedeuten.

2. Das Isomerengemisch 2,2-Dimethyl-3-cis-(2′,2′-dichlorvinyl)-cyclopropan-1R-carbonsäure-3-phenoxy-4-fluor-α(RS)-(2′-phenyl-äthinyl)-benzylester gemäss Anspruch 1.

3. 2,2-Dimethyl-3-cis-(2′,2′-dichlorvinyl)-cyclopropan-1R-carbonsäure-3-phenoxy-4-fluor-α(+)-(2′-phenyl-äthinyl)-benzylester gemäss Anspruch 2.

4. 2,2-Dimethyl-3-cis-(2′,2′-dichlorvinyl)-cyclopropan-1R-carbonsäure-3-phenoxy-4-fluor-α(-)-(2′-phenyl-äthinyl)-benzylester gemäss Anspruch 2.

5. Verfahren zur Herstellung der Verbindungen der Formel I, Anspruch 1, dadurch gekennzeichnet, dass man ein 3-cis-1R-Cyclopropancarbonsäurehalogenid der Formel II

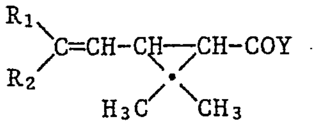

(II)

worin $R_1$ und $R_2$ die im Anspruch 1 angegebene Bedeutung haben und Y Halogen bedeutet, mit 3-Phenoxy-4-fluor-α-phenyläthinyl-benzylalkohol der Formel III

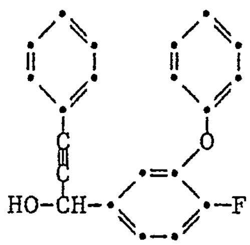

(III),

in Gegenwart einer Base umsetzt.

6. Der 3-Phenoxy-4-fluor-α-phenyläthinyl-benzylalkohol der Formel III.

7. Schädlingsbekämpfungsmittel, welches als aktive Komponente eine Verbindung gemäss einem der Ansprüche 1 bis 4 zusammen mit geeigneten Trägern und/oder anderen Zuschlagstoffen enthält.

8. Verwendung einer Verbindung gemäss einem der Ansprüche 1 bis 4 zur Bekämpfung von Insekten und Vertretern der Ordnung Akarina an Tieren und Pflanzen.

9. Verwendung gemäss Anspruch 8 zur Bekämpfung von pflanzenschädigenden Insekten.

10. Verwendung gemäss Anspruch 9 zur Bekämpfung von pflanzenschädigenden Insekten in Reiskulturen.

11. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss einem der Ansprüche 1 bis 4 oder mit einem Mittel enthaltend neben Zusatz-und Trägerstoffen eine pestizid wirksame Menge dieser Verbindung, in Kontakt bringt oder behandelt.

Europäisches Patentamt

# EUROPÄISCHER RECHERCHENBERICHT

Nummer der Anmeldung

EP 88 81 0666

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 190 096 (CIBA-GEIGY)<br>* Ansprüche *<br>--- | 1-11 | C 07 C 69/743<br>C 07 C 43/295<br>A 01 N 53/00 |
| D,A | EP-A-0 057 384 (BAYER)<br>* Ansprüche; Seite 4, Zeilen 15-21 *<br>--- | 1-11 | |
| A | FR-A-2 382 426 (BAYER)<br>* Ansprüche; Seite 32, Beispiel 12; Seite 33, Beispiel 17 *<br>----- | 1-4 | |

RECHERCHIERTE SACHGEBIETE (Int. Cl.4)

C 07 C 43/00
C 07 C 69/00

Der vorliegende Recherchenbericht wurde für alle Patentansprüche erstellt

| Recherchenort | Abschlußdatum der Recherche | Prüfer |
|---|---|---|
| DEN HAAG | 14-12-1988 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)